# EUROPÄISCHE PATENTSCHRIFT

(11) **EP 1 163 201 B1**
(45) Veröffentlichungstag und Bekanntmachung des Hinweises auf die Patenterteilung: **20.08.2003**
(21) Anmeldenummer: 00907643.1
(22) Anmeldetag: 28.02.2000
(51) Int. Cl.: C07C 57/05, C07C 57/07

(54) **VERFAHREN ZUR HERSTELLUNG VON ACRYLSÄURE**
METHOD FOR PRODUCING ACRYLIC ACID
PROCEDE DE PRODUCTION D'ACIDE ACRYLIQUE

(30) Priorität: 06.03.1999 DE 19909923; 28.05.1999 DE 19924533
(43) Veröffentlichungstag der Anmeldung: 19.12.2001
(73) Patentinhaber: BASF AKTIENGESELLSCHAFT, 67056 Ludwigshafen (DE)
(72) Erfinder: SCHRÖDER, Jürgen, D-67071 Ludwigshafen (DE); NESTLER, Gerhard, D-67061 Ludwigshafen (DE); MÜLLER-ENGEL, Klaus, Joachim, D-76297 Stutensee (DE)
(86) Internationale Anmeldenummer: EP0001635
(87) Internationale Veröffentlichungsnummer: WO00053560

(56) Entgegenhaltungen:
- WO-A-99/50219
- WO-A-99/50220
- DE-A- 4 101 879

## Beschreibung

Vorliegende Erfindung betrifft ein Verfahren zur Herstellung von Acrylsäure, bei dem man durch heterogen katalysierte Gasphasen-Partialoxidation von Propan oder Propen und/oder Acrolein mit molekularem Sauerstoff an im festen Aggregatzustand befindlichen Katalysatoren bei erhöhter Temperatur ein Acrylsäure enthaltendes heißes Produktgasgemisch erzeugt, die Temperatur des heißen, Acrylsäure enthaltenden Produktgasgemisches durch direkte Kühlung mit einer Quenchflüssigkeit verringert, den dabei nicht verdampfenden Teil der Quenchflüssigkeit 1, gegebenenfalls über einen indirekt kühlenden Wärmetauscher, im Kreis führt und als Auslaß einen Teil der nicht verdampften Quenchflüssigkeit 1 aus diesem Kreislauf ausschleust, anschließend das abgekühlte Produktgasgemisch in eine mit trennwirksamen Einbauten ausgerüstete Kolonne leitet, innerhalb der Kolonne in sich selbst aufsteigen läßt und dabei fraktioniert kondensiert, wobei im Seitenabzug eine rohe Acrylsäure und über den Kolonnensumpf und/oder über einen in Sumpfnähe gelegenen Seitenabzug Acrylsäure-Oligomere enthaltende Sumpf flüssigkeit und/oder Schwersiederfraktion entnommen und als Quenchflüssigkeit 1 verwendet wird.

Acrylsäure ist ein bedeutendes Zwischenprodukt, das beispielsweise im Rahmen der Herstellung von Polymerisatdispersionen Verwendung findet.

Unter anderem ist Acrylsäure durch heterogen katalysierte Gasphasen-Partialoxidation von Propan oder Propen und/oder Acrolein mit molekularem Sauerstoff an im festen Aggregatzustand befindlichen Katalysatoren bei erhöhter Temperatur erhältlich. Dabei werden die genannten Ausgangsgase, in der Regel mit inerten Gasen wie z.B. Stickstoff, CO₂ und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgasgemisch umgewandelt.

Durch Aufnahme in ein geeignetes Absorptionsmittel, z.B. Wasser oder ein Gemisch aus 70 bis 75 Gew.-% Diphenylether und 25 bis 30 Gew.-% Diphenyl, kann eine Grundabtrennung der Acrylsäure aus dem Produktgasstrom erzielt werden (vgl. z.B. EP-A 297445 und DE-PS 2136396).

Durch Entfernung des Absorptionsmittels über destillative Trennverfahren wird normalerweise eine Acrylsäure erhalten, die noch kein reines Produkt ist, weshalb sie normalerweise als rohe Acrylsäure oder auch Rohacrylsäure bezeichnet wird, die auf unterschiedliche Art und Weise weitergereinigt werden kann.

Nachteilig an den vorgenannten destillativen Trennverfahren ist jedoch, daß bei ihnen trotz einer Mitverwendung von radikalischen Polymerisationsinhibitoren in einem solchen Umfang die Bildung von in unerwünschter Weise radikalisch polymerisierter Acrylsäure einhergeht, daß die Destillationsvorrichtungen in nahezu periodischen Abständen (in der Regel einige Wochen) zum Zweck der Polymerisatentfernung abgeschaltet werden müssen.

Aus der DE-A 19740253, der DE-A 19740252, den nicht vorveröffentlichten DE-A's 19814421, 19814387 und 19814375 ist ein wie eingangs beschriebenes Verfahren zur Herstellung von Acrylsäure bekannt, bei dem die Grundabtrennung der Rohacrylsäure durch fraktionierte Kondensation des Produktgasgemisches der heterogen katalysierten Gasphasen-Partialoxidation vorgenommen wird. Eine solche Verfahrensweise ist auch Gegenstand der DE-A 19909923.

Als ein wesentlicher Vorteil der vorgenannten Grundabtrennung von Rohacrylsäure durch fraktionierte Kondensation hat es sich nun in überraschender und nicht verständlicher Weise herausgestellt, daß sie bei Mitverwendung von radikalischen Polymerisationsinhibitoren nur noch in deutlich geminderter Form von der Ausbildung radikalisch polymerisierter Acrylsäure gestört wird.

Nachteilig ist jedoch nach wie vor, daß in kondensierter Phase befindliche Acrylsäure durch Michael-Addition an sich selbst sowie an das sich dabei bildende Acrylsäure-Dimere Acrylsäure-Oligomere (Michael-Addukte) bildet (der Begriff Acrylsäure-Oligomere meint in dieser Schrift stets die entsprechenden Michael-Addukte und nicht durch radikalische Polymerisation entstehende Acrylsäureoligomere). Ein Beisein von Wasser, dem unvermeidbaren Nebenprodukt einer gasphasenkatalytisch oxidativen Herstellung von Acrylsäure, sowie erhöhte Temperaturen fördern die Bildung von Acrylsäure-Oligomeren.

Da Acrylsäure-Oligomere eine höhere Siedetemperatur als Acrylsäure aufweisen reichern sie sich sowohl im Rahmen einer destillativen Abtrennung von Acrylsäure als auch bei einer fraktionierten Kondensation des Produktgasgemisches einer gasphasenkatalytisch oxidativen Acrylsäureerzeugung in der Sumpfflüssigkeit an.

Prinzipiell ist eine Bildung von Acrylsäure-Oligomeren unerwünscht, da sie die Ausbeute an Wertprodukt mindert.

Es ist nun allgemein bekannt, daß die Bildung von Acrylsäure-Oligomeren ein reversibler Prozeß ist, der durch die Einwirkung erhöhter Temperaturen umgekehrt werden kann (vgl. z.B. DE-A 4101879, DE-A 19701737, DE-A 19536191, US-A 5734075, DE-A 2901783, US-A 3086046, US-A 2806878, DE-PS 1618129, Ullmanns Encyclopädie der technischen Chemie, 4. Auflage, Band 7, Verlag-Chemie, Weinheim, S. 83, rechte Spalte sowie Ullmann's Encyclopedia of Industrial Chemistry, Fifth, Completely Revised Edition, Verlag Chemie, Weinheim, Vol. A1, S. 167, rechte Spalte).

Diese thermische Rückspaltung kann sowohl sauer als auch basisch katalysiert oder auch in Abwesenheit von Katalysatoren durchgeführt werden (vgl. z.B. EP-A 717031).

Die Aufgabe der vorliegenden Erfindung bestand nun darin, in das wie eingangs beschriebene Verfahren zur Herstellung von Acrylsäure, bei dem die Grundabtrennung der Rohacrylsäure durch fraktionierte Kondensation des Produktgasgemisches der heterogen katalysierten Gasphasen-Partialoxidation durchgeführt wird, eine Rückspaltung der im Auslaß der Quenchflüssigkeit 1 enthaltenen Acrylsäure-Oligomeren so zu integrieren, daß die Laufzeit des Verfahrens, insbesondere jene der Kolonne zur fraktionierten Kondensation, im wesentlichen nicht gemindert wird.

Demgemäß wurde ein Verfahren zur Herstellung von Acrylsäure, bei dem man durch heterogen katalysierte Gasphasen-Partialoxidation von Propan oder Propen und/oder Acrolein mit molekularem Sauerstoff an im festen Aggregatzustand befindlichen Katalysatoren bei erhöhter Temperatur ein Acrylsäure enthaltendes heißes Produktgasgemisch erzeugt, die Temperatur des heißen, Acrylsäure enthaltenden Produktgasgemisches durch direkte Kühlung mit einer Ouenchflüssigkeit 1 verringert, den dabei nicht verdampfenden Teil der Quenchflüssigkeit 1, gegebenenfalls über einen indirekt kühlenden Wärmetauscher, im Kreis führt und als Auslaß einen Teil der nicht verdampften Quenchflüssigkeit 1 aus diesem Kreislauf ausschleust, anschließend das abgekühlte Produktgasgemisch in eine mit trennwirksamen Einbauten ausgerüstete Kolonne leitet, innerhalb der Kolonne in sich selbst aufsteigen läßt und dabei fraktioniert kondensiert, wobei im Seitenabzug eine rohe Acrylsäure und über den Kolonnensumpf und/oder über einen in Sumpfnähe gelegenen Seitenabzug Acrylsäure-Oligomere enthaltende Sumpfflüssigkeit und/oder Schwersiederfraktion (schwerer flüchtig als Acrylsäure) entnommen und als Quenchflüssigkeit 1 verwendet wird, gefunden, das dadurch gekennzeichnet ist, daß man den Auslaß der Quenchflüssigkeit 1 einem Spaltgefäß zuführt und in selbigem die im Auslaß der Quenchflüssigkeit 1 enthaltenen Acrylsäure-Oligomeren bei erhöhter Temperatur in Acrylsäure rückspaltet, dabei gasförmig aus der Flüssigphase entweichende Acrylsäure kondensiert und das resultierende Kondensat in den Kreislauf der Quenchflüssigkeit 1 einspeist.

Die Einleitstelle des gequenchten Produktgasgemisches der katalytischen Gasphasenoxidation in die mit trennwirksamen Einbauten ausgerüstete Kolonne befindet sich in zweckmäßiger Weise unterhalb der trennwirksamen Einbauten. Prinzipiell kann die fraktionierte Kondensation innerhalb der Kolonne in an sich bekannter Weise durch indirekte Kühlung und/oder Erwärmung bewirkt werden. Es ist jedoch zweckmäßiger die fraktionierte Kondensation wie folgt zu bewirken. Über einen oberhalb der Einleitstelle und unterhalb der Einbauten angebrachten ersten Fangboden wird ein Teil der beim Aufsteigen des gequenchten Produktgasgemisches sich bildenden und/oder kondensierenden, höher als Acrylsäure siedenden, Substanzen entnommen. Ein Teil der entnommenen Schwersiederfraktion wird im Gemisch mit der der Kolonne entnommenen Sumpfflüssigkeit als Quenchflüssigkeit 1 verwendet und die verbleibende Teilmenge der entnommenen Schwersiederfraktion wird in einem indirekten Wärmetauscher abgekühlt oder erwärmt und oberhalb des ersten, aber unterhalb eines in der unteren Kolonnenhälfte angebrachten zweiten Fangbodens in die Kolonne rückgeführt. Über den zweiten Fangboden wird im Seitenabzug als Mittelsiederfraktion die Rohacrylsäure entnommen, die im Normalfall eine Reinheit ≥ 95 Gew.-% aufweist. Zweckmäßigerweise wird man die Rohacrylsäure weiteren destillativen und/oder kristallisativen Reinigungsstufen zuführen und wenigstens einen Teil der im Rahmen der Destillationen und/oder Kristallisationen anfallenden Sumpfflüssigkeiten und/oder Mutterlaugen unterhalb des zweiten, aber oberhalb des ersten Fangbodens in die Kolonne rückführen. Alternativ dazu kann die Rückführung der Sumpfflüssigkeiten und/oder Mutterlaugen auch so erfolgen, daß sie in zwei Teilströme aufgeteilt werden, von denen einer unterhalb des zweiten, aber oberhalb des ersten Fangbodens und der andere oberhalb des zweiten Fangbodens in die Kolonne rückgeführt wird. Der letztgenannte der beiden Teilströme wird in der Regel, bezogen auf die Gesamtrückführung, bis zu 35 Gew.-% betragen. Aus dem am Kopf der Kolonne entweichenden Leichtsiedergasstrom wird man zweckmäßig durch direkte Kühlung in einem an Einbauten freien oder Einbauten enthaltenden Raum mittels einer zweiten Quenchflüssigkeit (in dieser Schrift zum Zweck der Differenzierung als Quenchflüssigkeit 2 bezeichnet) im wesentlichen Wasser sowie schwerer als Wasser flüchtige Bestandteile auskondensieren. Das dabei gewonnene Kondensat wird als Sauerwasser bezeichnet. Einen Teil des Sauerwassers wird man in sinnvoller Weise zur Erhöhung der Trennleistung am Kopf der Kolonne in selbige rückführen. Ein weiterer Teil des Sauerwassers wird zweckmäßigerweise ausgeschleust und entsorgt (z.B. verbrannt) und der verbleibende Teil des Sauerwassers wird üblicherweise in einem externen Wärmetauscher indirekt abgekühlt und als Quenchflüssigkeit 2 verwendet.

Leichter als Wasser flüchtige Bestandteile des Leichtsiederstroms werden im wesentlichen gasförmig abgezogen und gegebenenfalls als Verdünnungsgas in die Gasphasenoxidation rückgeführt.

Alternativ kann man den Sauerwasserquench in die Kolonne für die fraktionierte Kondensation integrieren. In diesem Fall wird über einen weiteren Fangboden im oberen Teil der Kolonne wäßrige Rücklaufflüssigkeit entnommen, in einem Wärmetauscher indirekt gekühlt und bis auf den zu entsorgenden Auslaßanteil teilweise am Kopf der Kolonne und teilweise unterhalb des Fangbodens rückgeführt. Das gegebenenfalls in die Gasphasenoxidation rückzuführende Abgas verläßt die Kolonne in diesem Fall an deren Kopf.

Der Quench des Produktgasgemisches der heterogen katalysierten Gasphasen-Partialoxidation mit der Quenchflüssigkeit 1 wird in der Regel in einem an Einbauten freien Raum ausgeführt. Die dabei Kühlgrenztemperatur erreichende Quenchflüssigkeit 1 wird, gegebenenfalls über einen externen Wärmetauscher, im Kreis geführt und zur Direktkühlung wiederverwendet. Ein Teil der Quenchflüssigkeit 1 wird aus diesem Kreislauf als Auslaß ausgeschleust und der erfindungsgemäß zu integrierenden Rückspaltung zugeführt.

Der Reinheitsgrad der über Seitenabzug aus der fraktionierten Kondensationskolonne entnommenen Rohacrylsäure beträgt üblicherweise ≥ 95 Gew.-%. D.h., die Rohacrylsäure enthält zu wenigstens 95 % ihres Gewichtes Acrylsäuremoleküle.

Die Polymerisationsinhibierung des erfindungsgemäßen Verfahrens kann im wesentlichen wie in der DE-A 19909923 beschrieben durchgeführt werden. D.h., sie kann beispielsweise dadurch erfolgen, daß am Kopf der Kondensationskolonne Phenothiazin oder ein Gemisch aus einem N-Oxyl-Radikal und einer Benzolverbindung, die zwei über Heteroatome an den aromatischen Ring gebundene Substituenten und wenigstens einen beweglichen Wasserstoff aufweist, zugesetzt wird.

Eine günstigere Form der Polymerisationsinhibierung ist dadurch gekennzeichnet, daß sie ausschließlich mittels N-Oxyl-Radikalen (z.B. die in der EP-A 765856 genannten) erfolgt. Das sind Verbindungen, die wenigstens eine Gruppe -N-O• aufweisen.

Erfindungsgemäß bevorzugte N-Oxyl-Radikale sind die Pyrrolidin-1-oxyl-Typen und die Piperidin-1-oxyl-Typen. Beispielhaft genannt seien 4,4',4"-Tris-(2,2,6,6-tetramethylpiperidin-1-oxyl)phosphit, 2,2,6,6-Tetramethylpiperidin-1-oxyl (TEMPO), 4-Hydroxy-2,2,6,6-tetramethylpiperidin-1-oxyl (4-OH-TEMPO), 4-Oxo-2,2,6,6-tetramethylpiperidin-1-oxyl (4-Oxo-TEMPO), 4-Dimethylamino-2,2,6,6-tetramethylpiperidin-1-oxyl, 4-Amino-2,2,6,6-tetramethylpiperidin-1-oxyl, 4-Ethanoyloxy-2,2,6,6-tetramethylpiperidin-1-oxyl, 2,2,5,5-Tetramethylpyrrolidin-1-oxyl und 3-Amino-2,2,5,5-tetramethylpyrrolidin-1-oxyl. Die N-Oxyl-Inhibitoren werden vorzugsweise als 0,1 bis 2 gew.-%ige Lösungen in Wasser und/oder in Sauerwasser eingesetzt. Die Zugabe der wäßrigen N-Oxyl-Inhibitorlösung erfolgt zweckmäßigerweise im oberen Viertel der Kondensationskolonne und/oder in den Sauerwasserquench. Die wäßrige Lösung kann nur ein N-Oxyl-Radikal oder ein Gemisch von N-Oxyl-Radikalen enthalten. Eine ausreichende Inhibierung kann bereits durch Zugabe einer wäßrigen Lösung erzielt werden, die ausschließlich 4-OH-TEMPO als Polymerisationsinhibitor enthält.

Die Zugabemenge der einzusetzenden N-Oxyl-Inhibitoren wird in zweckmäßiger Weise so bemessen, daß die aus der Kolonne entnommene Schwersiederfraktion und Sumpfflüssigkeit 1 bis 1000 gew.ppm, bezogen auf das Gewicht der Schwersiederfraktion, an N-Oxyl-Inhibitoren enthält. Da die aus der Kolonne entnommene Schwersiederfraktion und Sumpfflüssigkeit als Quenchflüssigkeit 1 dient, wird das Quenchsystem 1 automatisch mitstabilisiert. Bei Bedarf kann das Quenchsystem 1 durch Zusatz einer Phenothiazinverbindung co-stabilisiert werden. Als solche Phenothiazinverbindungen kommen beispielsweise Phenothiazin (PTZ) selbst, Bis-(α-methylbenzyl)phenothiazin, 3,7-Dioctylphenothiazin und Bis-(α-dimethylbenzyl)phenothiazin in Betracht, unter denen das Phenothiazin bevorzugt ist. Letzteres gilt insbesondere dann, wenn 4-OH-TEMPO zur Stabilisierung der Kondensationskolonne mitverwendet oder ausschließlich verwendet wird. Ein solcher Phenothiazinzusatz kann, bezogen auf das Gewicht der Quenchflüssigkeit, 1 bis 500 gew.ppm betragen. Zweckmäßigerweise erfolgt ein solcher Zusatz einer Phenothiazinverbindung in Acrylsäure, vorzugsweise in über Seitenabzug entnommener Rohacrylsäure gelöst (typisch 0,1 bis 2 gew.-%ig).

Der zur Polymerisationsinhibierung erforderliche Bedarf an N-Oxyl-Radikalen läßt sich dadurch absenken, daß die weniger anspruchsvolle Sauerwasserstabilisierung, d.h., die Stabilisierung des Quenchsystems 2, ersatzweise oder in Kombination mit einer wäßrigen Lösung wenigstens einer Benzolverbindung, die zwei über Heteroatome an den aromatischen Ring gebundene Substituenten und wenigstens einen beweglichen Wasserstoff aufweist (z.B. die in der EP-A 766856 genannten), z.B. einer Phenolverbindung, durchgeführt wird (typisch 0,1 bis 2 gew.-%ige Lösung). Als solche Phenolverbindungen kommen beispielsweise Hydrochinon oder Methochinon (p-Methoxyphenol = MEHQ) in Betracht, unter denen letztere bevorzugt ist. Dies gilt insbesondere dann, wenn im Kopfbereich der Kolonne ausschließlich 4-OH-TEMPO eingesetzt und das Quenchsystem mit PTZ co-stabilisiert wird. In der Regel werden, bezogen auf das Gewicht des Sauerwassers, 1 bis 500 gew.ppm wenigstens einer Phenolverbindung für eine solche Sauerwasserinhibierung verwendet.

Eine alternative Polymerisationsinhibierung besteht darin, am Kopf der Kondensationskolonne eine wäßrige Lösung von MEHQ aufzugeben und die Sauerwasserinhibierung ebenfalls durch Zusatz einer Lösung von MEHQ in Wasser und/oder in Sauerwasser durchzuführen. Zusätzlich wird im Mittelteil der Kondensationskolonne sowie gegebenenfalls im Produktgasgemischquench (Quench 1) eine Lösung von PTZ in Acrylsäure (z.B. Rohacrylsäure) aufgegeben.

Wie bereits erwähnt, wird das Quenchsystem 1 über die Stabilisierung der Kondensationskolonne automatisch mitstabilisiert und kann bei Bedarf durch Zusatz von Phenothiazin und/oder Metochinon co-stabilisiert werden.

Die dabei erzielte Polymerisationsinhibierung der Quenchflüssigkeit 1 ist in der Regel auch ausreichend, um bei der erfindungsgemäß zu integrierenden Rückspaltung eine ausreichende Stabilität des Auslaß der Quenchflüssigkeib 1 gegen unerwünschte Polymerisatbildung zu gewährleisten. Hingegen werden bei der Kondensation der im Rahmen der Rückspaltung gasförmig entweichenden, Acrylsäure enthaltenden, Dämpfe die Kondensatoroberflächen zweckmäßigerweise extra inhibiert (die in der Quenchflüssigkeit 1 enthaltenen Inhibitoren verdampfen in der Regel nicht mit). Diese Polymerisationsinhibierung der Kondensatoroberflächen wird man mit Vorteil mit denselben Polymerisationsinhibitoren vornehmen, die auch zur Inhibierung der Kondensationskolonne verwendet und/oder empfohlen wurden.

Verwendet man zur Stabilisierung der Kondensationskolonne und_des Sauerwasserquench z.B. ausschließlich 4-OH-TEMPO, so ist es sinnvoll, die Kondensatoroberflächen auch ausschließlich mit 4-OH-TEMPO, zweckmäßig in Rohacrylsäure gelöst, vorzunehmen. Selbstverständlich können die Kondensatoroberflächen aber auch mittels PTZ, MEHQ und/oder Hydrochinon co-stabilisiert oder ausschließlich stabilisiert werden.

In der Regel wird die erfindungsgemäß zu integrierende Rückspaltung bei einem Druck von ≤ 1 bar und bei einer Temperatur von 130 bis 250°C durchgeführt. Mit Vorteil beträgt der Druck für die Rückspaltung 25 bis 600, vorzugsweise 100 bis 300 mbar. Die Rückspalttemperatur liegt zweckmäßigerweise bei 140 bis 230°C, vorzugsweise bei 160 bis 200°C. Wird die Rückspaltung kontinuierlich durchgeführt (das erfindungsgemäße Verfahren wird vorzugsweise kontinuierlich durchgeführt), so sollte die Verweilzeit im Rückspaltreaktor etwa 0,5 bis 3 h betragen. In einfacher Weise läßt sich die erfindungsgemäß zu integrierende Rückspaltung in einem beheizbaren Rührreaktor durchführen. Wie in der US-A 5734075 sowie in der DE-A 4101879 beschrieben, läßt sich die Rückspaltung der im Auslaß des Quenchsystems 1 enthaltenen Acrylsäure-Oligomere ohne Zusatz spezieller saurer oder basischer Spaltkatalysatoren durchführen. Mit Vorteil wird man die Rückspaltung jedoch im Beisein von Spaltkatalysatoren durchführen. Als solche kommen z.B. Dodecylbenzolfulfonsäure, p-Toluolsulfonsäure, Schwefelsäure oder die festen sauren Katalysatoren der JP-A 3-178949 in Betracht.

Insbesondere im Fall einer Polymerisationsinhibierung mittels N-Oxyl-Radikalen, vor allem wenn 4-OH-TEMPO als alleiniger Polymerisationsinhibitor oder als co-Stabilisator des Quenchsystems 1 verwendet wird, ist es zweckmäßig die Rückspaltung durch Zusatz eines anorganischen Salzes, dessen Zusatz zu einer wäßrigen Lösung einer starken Brönsted-Säure den pH-Wert der wäßrigen Lösung ins alkalische verschiebt, vorzunehmen, wie es z.B. die DE-C 2407236 empfiehlt. Bezogen auf die der Rückspaltung zu unterwerfende Menge an Auslaß der Quenchflüssigkeit 1 wird die zuzusetzende Menge an basischem Rückspaltkatalysator in der Regel 0,1 bis 5 Gew.-% betragen. Beispiele für erfindungsgemäß geeignete Rückspaltkatalysatoren sind KOH, K₂CO₃, KHCO₃, NaOH, Na₂CO₃, NaHCO₃, LiOH, Li₂CO₃ und CaCO₃.

D.h., geeignete Rückspaltkatalysatoren sind insbesondere die Alkali- und/oder Erdalkalisalze von schwachen anorganischen oder organischen Brönstedsäuren wie z.B. Phosphorsäure, Borsäure, Kohlensäure, Ameisensäure oder Essigsäure. Mit anderen Worten eignen sich somit als Rückspaltkatalysatoren vor allem Alkali- und/oder Erdalkaliphosphate, -borate, -carbonate, -hydrogencarbonate, -formiate und -acetate.

Vorzugsweise wird man die Rückspaltkatalysatoren so wählen, daß sie unter den Rückspaltbedingungen im Auslaß der Quenchflüssigkeit 1 löslich sind. Gemäß US-A 4293347 wirkt sich auch ein Beisein von Dialkylphthalaten vorteilhaft auf die relevante Rückspaltung aus.

Der im Spaltreaktor verbleibende schwerflüchtige Rückstand wird beim erfindungsgemäßen Verfahren regelmäßig seiner Entsorgung, z.B. seiner Verbrennung, zugeführt. Dazu kann der Spaltrückstand gegebenenfalls mit hydrophilen organischen Flüssigkeiten wie organischen Säuren (z.B. Ethylhexansäure, Propionsäure) oder Rückstände aus der Herstellung dieser Säuren aber auch mit Alkanolen wie Ethanol oder Methanol verdünnt werden.

An dieser Stelle sei noch festgehalten, daß der im erfindungsgemäßen Verfahren zu verwendenden Quenchflüssigkeit 1 bei Bedarf eine höher als Acrylsäure siedende inerte organische Flüssigkeit zugesetzt werden kann, die die Quenchflüssigkeit 1 fluid hält.

Als solche hochsiedenden inerten organischen Flüssigkeiten kommen insbesondere alle diejenigen in Betracht, die in der DE-A 2136396 und in der DE-A 4308087 empfohlen werden. Dies sind im wesentlichen Flüssigkeiten, deren Siedepunkt bei Normaldruck oberhalb von 160°C liegt. Beispielhaft genannt seien Ethylhexansäure, N-Methylpyrrolidon, Mittelölfraktionen aus der Paraffindestillation, Diphenylether, Diphenyl oder Mischungen der vorgenannten Flüssigkeiten wie z.B. ein Gemisch aus 70 bis 75 Gew.-% Diphenylether und 25 bis 30 Gew.-% Diphenyl. Günstig sind die Verwendung eines Gemisches bestehend aus einer Mischung aus 70 bis 75 Gew.-% Diphenylether und 25 bis 30 Gew.-% Diphenyl, sowie, bezogen auf diese Mischung, 0,1 bis 25 Gew.-% o-Dimethylphthalat.

Im vorgenannten Fall wird bei der Rückspaltung wenigstens eine Teilmenge der mitverwendeten inerten organischen Flüssigkeit mitverdampfen. Verbleibt eine Teilmenge der organischen Flüssigkeit im Spaltrückstand, kann selbiger einer Aufarbeitung zugeführt werden, in der das mitverwendete Lösungsmittel, z.B. destillativ, abgetrennt und in den Quench 1 rückgeführt wird. Die verbleibenden Schwersieder werden entsorgt.

Häufig sind die heißen Produktgasgemische der heterogen katalysierten Gasphasenoxidation wie folgt zusammengesetzt:
1 bis 30 Gew.-% Acrylsäure,
0,01 bis 3 Gew.-% Essigsäure,
0,01 bis 1 Gew.-% Propionsäure,
0,01 bis 0,5 Gew.-% Maleinsäure/Maleinsäureanhydrid,
0,05 bis 1 Gew.-% Acrolein,
0,05 bis 1 Gew.-% Formaldehyd,
0,1 bis 1 Gew.-% Furfural,
0,01 bis 0,5 Gew.-% Benzaldehyd,
0,01 bis 1 Gew.-% Propen,
0,05 bis 10 Gew.-% Sauerstoff,
1 bis 30 Gew.-% Wasser und
als Restmenge inerte Gase wie z.B. Stickstoff, Kohlenoxide, Methan und Propan.

Die Gasphasenoxidation selbst kann z.B. in zwei aufeinanderfolgenden Oxidationsstufen durchgeführt werden, wie sie in der EP-A 700 714 und in der EP-A 700 893 beschrieben sind. Selbstverständlich können aber auch die in der DE-A 19740253 sowie die in der DE-A 19740252 zitierten Gasphasenoxidationen zur Anwendung kommen.

In der Regel beträgt die Temperatur des die Gasphasenoxidation verlassenden Produktgasgemisches 150 bis 350°C, meist 200 bis 300°C. Im Quenchsystem 1 wird das heiße Produktgasgemisch üblicherweise auf eine Temperatur von 100 bis 180°C abgekühlt, mit der es dann in den untersten Abschnitt der Trennkolonne geführt wird. Der in der Kolonne herrschende Betriebsdruck beträgt in der Regel 0,5 bis 5 bar, häufig 0,5 bis 3 bar und vielfach 0,5 bis 2 bar.

Als Quenchvorrichtung 1 können alle im Stand der Technik für diesen Zweck bekannten Vorrichtungen (z.B. Sprühwäscher, Venturiwäscher, Blasensäulen oder sonstige Apparate mit berieselten Oberflächen) eingesetzt werden, wobei erfindungsgemäß vorzugsweise Venturi-Wäscher oder Sprühkühler verwendet werden.

Zur indirekten Kühlung oder Erwärmung der Quenchflüssigkeit 1 eignen sich alle gängigen Wärmeüberträger oder Wärmetauscher. Als bevorzugt seien Rohrbündelwärmetauscher, Plattenwärmetauscher und Luftkühler genannt. Die Temperatur der Quenchflüssigkeit 1 beträgt nach Verlassen des Wärmetauschers normalerweise 70 bis 200°C, häufig 100 bis 150°C. Geeignete Kühlmedien sind Luft beim entsprechenden Luftkühler und Kühlflüssigkeiten, insbesondere Wasser, bei den anderen Kühlvorrichtungen. Als Kolonneneinbauten kommen prinzipiell alle gängigen Einbauten in Betracht, insbesondere Böden, Packungen und/oder Füllkörper. Von den Böden sind Glockenböden, Siebböden, Ventilböden und/oder Dual-Flow-Böden bevorzugt. In typischer Weise beträgt bei einer Bodenkolonne die Gesamtzahl an Trennböden 20 bis 80 (für den Fall, daß der Sauerwasserquench in die Kolonne integriert ist bis 100), bevorzugt 50 bis 80 (bzw. 100). Die im Seitenabzug entnommene Rohacrylsäure enthält in der Regel noch

| | |
|---|---|
| 0,1 bis 2 Gew.-% | Essigsäure, |
| 0,5 bis 5 Gew.-% | Wasser, |
| 0,05 bis 1 Gew.-% | niedermolekulare Aldehyde, |
| 0,001 bis 1 Gew.-% | Maleinsäure und/oder deren Anhydrid sowie |
| 1 bis 500 Gew.ppm | Polymerisationsinhibitor, |

jeweils bezogen auf das Gewicht der Rohacrylsäure. Die Temperatur im Sumpf der Kolonne liegt in typischer Weise bei 90 bis 130°C, wohingegen die Kopftemperatur normalerweise 40 bis 80, häufig 50 bis 70°C beträgt. Die Entnahmetemperatur der Rohacrylsäure liegt meist bei 80 bis 110°C. Die Rückführtemperatur der Schwersieder beträgt beim Eintritt in die Kolonne in typischer Weise 95 bis 115°C. Die Rückführtemperatur des Sauerwassers in die Kolonne liegt in der Regel bei 25 bis 35°C. Prinzipiell kann das Quenchsystem 2 wie das Quenchsystem 1 gestaltet werden. Selbstverständlich kann erfindungsgemäß die Entnahme der Rohacrylsäure auch über mehrere in kurzen Abständen aufeinanderfolgende Fangböden vorgenommen werden.

Die als Mittelsiederfraktion entnommene Rohacrylsäure kann erfindungsgemäß zum Zwecke der Weiterreinigung z.B. einer Kristallisation zugeführt werden. Hierbei wird in der Regel ohne Zusatz eines Lösungsmittels, insbesondere ohne Zusatz eines organischen Lösungsmittels gearbeitet. Das zu verwendende Kristallisationsverfahren unterliegt keiner Beschränkung. Die Kristallisation kann kontinuierlich oder diskontinuierlich, einstufig oder mehrstufig bis zu nahezu beliebigen Reinheitsgraden durchgeführt werden. Bei Bedarf kann der kristallisativ zu reinigenden Rohacrylsäure vorab einer Kristallisation Wasser zugesetzt werden (bezogen auf die enthaltene Menge an Acrylsäure bis zu 10 Gew.-% oder mehr, vorzugsweise bis zu 5 Gew.-%). Ein solcher Zusatz erleichtert die Abtrennung von in der Rohacrylsäure als Nebenprodukt enthaltener Essigsäure, da diese im Beisein von Wasser in geringerem Ausmaß in die Acrylsäurekristalle eingebaut wird. Außerdem mindert ein Beisein von Wasser die Verkrustungsneigung.

Es überrascht, daß eine veresterungsgerechte Rohacrylsäure bereits durch eine einzige Kristallisationsstufe erzielt werden kann. Zweckmäßigerweise wird diese Kristallisationsstufe als Suspensionskristallisation ausgeführt. Als Kristaller wird dazu vorteilhaft ein Trog verwendet, in welchem gewischte Kühlplatten (die innerlich von einem Kühlmedium durchflossen werden) hintereinander hängend angeordent sind. Durch das Wischen der Kühlplatten wird die Ausbildung einer Schichtkristallisation unterdrückt. Die Rohacrylsäure wird von hinten nach vorne kontinuierlich durch den Kristaller geführt (gepumpt oder überlaufgeregelt). Die einphasige Rohacrylsäure verdickt dabei zu einer zweiphasigen, Acrylsäurekristalle als feste Phase enthaltenden, Suspension. Ein diesbezüglich besonders geeigneter Kristaller ist ein Kristaller der Fa. GMF Gouda (Holland) vom Typ Kühlscheibenkristallisator (Cooling Disc Crystallizer). In zweckmäßiger Weise werden die gebildeten Kristalle aus der vorgenannten Suspension mittels einer Zentrifuge abgetrennt (z.B. eine solche der Fa. Siebtechnik, vom Typ Schubzentrifuge SHS mit konischer Siebtrommel) und bei Bedarf mit bereits weiter gereinigter Rohacrylsäure gewaschen und/oder einem später beschriebenen Schwitzen unterworfen. Das Abtrennen und Waschen der Suspensionskristalle kann aber auch vorteilhaft in einer Waschkolonne durchgeführt werden, wie es z.B. in der EP-A 97405, US-A 3872009, EP-A 98637, EP-A 305316, EP-A 105524 und der WO 84/00118 beschrieben ist. Dann werden die Kristalle üblicherweise in einen Behälter gegeben, der vorteilhaft bereits eine Menge aufgeschmolzener, in entsprechender Weise gereinigter, Acrylsäurekristalle enthält. Bei Bedarf enthält diese aufgeschmolzene Acrylsäure zusätzlichen Polymerisationsinhibitor zugesetzt (z.B. MEHQ, PTZ oder 4-Hydroxy-TEMPO). In der Regel genügt jedoch der in den Kristallen verbliebene Inhibitorrest, um eine ausreichende Inhibierung zu gewährleisten. Durch indirektes Erwärmen werden anschließend die gewonnenen Acrylsäurekristalle aufgeschmolzen. Die so erhältliche Acrylsäureschmelze weist normalerweise eine Reinheit ≥98 Gew. -% auf und kann unmittelbar als veresterungsgerechte Acrylsäure vermarktet werden.

Anstelle einer Suspensionskristallisation kann auch eine Schichtkristallisation, z.B. eine Fallfilmkristallisation, angewendet werden, wie sie z.B. in der EP-A 616 998. zur Gewinnung von Reinacrylsäure beschrieben ist. Als flüssiger Kälte-/Wärmeträger kommen dabei z.B. Wasser/Methanol, Wasser/Ethanol- und Wasser/Ethylenglycolmischungen in Betracht.

Zur Gewinnung besonders hoher Reinheitsgrade ("Reinacrylsäure") wird die Kristallisation zweckmäßigerweise als fraktionierte Kristallisation durchgeführt. Üblicherweise werden bei fraktionierter Kristallisation alle Stufen, die ein Kristallisat erzeugen, das reiner ist als die zugeführte Schmelze, Reinigungsstufen genannt. Alle anderen Stufen heißen Abtriebsstufen. Zweckmäßigerweise werden mehrstufige Verfahren nach dem Gegenstromprinzip betrieben, bei dem nach der Kristallisation in jeder Stufe das Kristallisat von der Mutterlauge abgetrennt und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Vorteilhafterweise liegt die Temperatur der Flüssigphase während der Kristallisation zwischen +5 und + 14°C, insbesondere zwischen +8°C und +12°C. Der Feststoffgehalt im Kristallisator liegt vorteilhafterweise zwischen 0 und 80 g Feststoff/100 g Gesamtmasse. Bei der Suspensionskristallisation beträgt der Feststoffgehalt vorzugsweise zwischen 15 und 35 g Feststoff/100 g Gesamtmasse und bei der Schichtkristallisation 50 bis 80 g Feststoff/100 g Gesamtmasse.

In einer möglichen Ausgestaltung der Erfindung erfolgt die Kristallisation durch Kühlung von Apparatewänden und/oder durch Verdampfung der Lösung im Vakuum. Bei der Kristallisation durch Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere Ausführungsform bei der Kühlungskristallisation ist die Verwendung von Kühlscheibenkristallern, wie sie z.B. von der Fa. Gouda (Holland) hergestellt werden. Bei einer weiteren geeigneten Variante zur Kristallisation durch Kühlung wird die Wärme über herkömmliche Wärmeüberträger (bevorzugt Rohrbündel oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den Wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit aufgeschmolzenen Kristallen). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen. In einer weiteren Ausführung der Erfindung erfolgt die Kristallisation in Apparaten, in denen die Kristalle im Kristallisationsapparat an gekühlten Flächen aufwachsen, d.h. im Apparat fixiert sind (z.B. Schichtkristallisationsverfahren (vgl. z.B. EP-A 616998) der Fa. Sulzer Chemtech (Schweiz) oder statisches Kristallisationsverfahren, (vgl. z.B. FR-A 2668946) der Fa. BEFS PROKEM (Frankreich)).

Die erhaltenen Acrylsäurekristalle werden wie bereits erwähnt von der verbliebenen Mutterlauge abgetrennt. Für den Fall der Schichtkristallisation oder der statischen Kristallisation kann die Trennung der Kristalle von der Mutterlauge im Kristallisationsapparat selbst erfolgen, da die Kristalle im Apparat fixiert sind und die Mutterlauge durch Abfließenlassen aus dem Apparat entfernt werden kann. Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. In einer bevorzugten Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Beim Waschen liegt die Waschflüssigkeitsmenge geeigneterweise zwischen 0 und 500 g Waschflüssigkeit/100 g Kristallisat, vorzugsweise zwischen 30 und 200 g Waschflüssigkeit/100 g Kristallisat. Die verwendete Waschflüssigkeit unterliegt keiner Einschränkung. Vorteilhafterweise wird jedoch mit Reinprodukt gewaschen, d.h. mit einer Flüssigkeit, die Acrylsäure enthält, deren Reinheit gleich oder höher ist als die des zu waschenden Kristallkuchens. Daneben ist auch eine Wäsche mit Wasser möglich. Das Waschen kann in hierfür üblichen Apparaten erfolgen.

Vorteilhafterweise werden Waschkolonnen (z.B. solche, mit mechanischem Betttransport (z.B. von der Fa. Niro Process Technology B.V., in s'Hertogenbusch (NL)) oder solche mit hydraulischem Betttransport (z.B. von der Firma TNO in Apeldoorn (NL))), in denen die Abtrennung der Mutterlauge und das Waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche. Vorteilhafterweise beträgt die Schwitzmenge zwischen 0 und 80 g abgeschmolzenes Kristallisat/100 g Kristallisat vor dem Schwitzen, vorzugsweise zwischen 5 und 35 g abgeschmolzenes Kristallisat/100 g Kristallisat. Besonders bevorzugt ist die Durchführung des Schwitzens auf Zentrifugen oder Bandfiltern sowie in Kristallern, in denen die Kristalle im Kristaller fixiert sind (z.B. Schichtkristaller). Auch die Durchführung einer Kombination aus Waschen und Schwitzen in einem Apparat kann geeignet sein.

Die Acrylsäurekristalle nach der Fest-Flüssig-Trennung und ggf. weiterem Waschen und/oder Schwitzen stellen die gereinigte Säure aus dem Verfahren dar. Die Reinheit der erhaltenen Kristalle beträgt in der Regel 97 bis 99,99 Gew.-% und mehr Acrylsäure, insbesondere 98,5 bis 99,9 Gew.-% Acrylsäure. Die nach dem erfindungsgemäßen Verfahren hergestellten Kristalle enthalten nurmehr ganz geringe Mengen an Verunreinigungen, wie Essigsäure, Propionsäure und/oder Diacrylsäure.

Zusammenfassend sei nochmals festgehalten, daß die Kristallisation erfindungsgemäß prinzipiell als Suspensionskristallisation und/oder als Schichtkristallisation (die abgeschiedenen Kristalle verbleiben im Kristaller fixiert) realisiert werden kann. Als letztere Kristallisationsmethode kommt z.B. die Fallfilmkristallisation (z.B. wie in EP-A 616 998 beschrieben), die Kristallisation im volldurchströmten Rohr (z.B. gemäß DE-A 2606364) oder eine statische Kristallisation in Betracht.

Bei der Suspensionskristallisation kann die Kühlung direkt (z.B. Verdampfung im Vakuum) und/oder indirekt-mittels gekühlter Flächen redisiert werden. Als Kristaller kommen für eine solche Suspensionskristallisation in Betracht: Rührkessel mit wandgängigen Rührern, Kratzkühler, Kühlscheibenkristaller der Fa. Gouda, sowie Umlaufkristallisation mit Wärmeüberträgern ohne Vorrichtung zur Vermeidung von Kristallschichtbildung.

Falls gewünscht, kann die gereinigte Säure nach bekannten Methoden verestert oder nach bekannten Methoden weiter gereinigt werden.

Zur Erhöhung der Ausbeute an Acrylsäure wird ganz generell die nach Beendigung der Kristallisation verbliebene Mutterlauge wenigstens teilweise, wie eingangs beschrieben, in die Trennkolonne rückgeführt. Der Anteil der rückgeführten Mutterlauge liegt, bezogen auf ihre anfallende Menge, erfindungsgemäß bei > 0 bis 100 Gew.-%, vorzugsweise bei 80 bis 100 Gew.-%. Über die Rückführung der Mutterlauge wird gleichzeitig die Rückführung des bei der Kristallisation abgetrennten Polymerisationsinhibitors gewährleistet. Das erfindungsgemäße Verfahren gewährleistet eine voll befriedigende Polymerisationsinhibierung bei minimiertem Einsatz an Polymerisationsinhibitoren.

Bei Bedarf kann erfindungsgemäß zusätzlich zum Produktgemisch noch in begleitender Weise molekularer Sauerstoff oder ein molekularen Sauerstoff enthaltender Inertgasstrom durch die Trennkolonne geführt werden. Dies verstärkt die Wirkung der zugesetzten Polymerisationsinhibitoren.

In entsprechender Weise wie hier beschrieben, läßt sich selbstredend das zum beschriebenen Verfahren äquivalente Verfahren zur Herstellung von Methacrylsäure inhibieren und betreiben.

Mögliche Ausgangsverbindungen für die Gasphasenoxidation sind dabei Isobuten, Methyl-tert.butylether, Isobutan, Isobuttersäure, tert. Butanol, Isobutyraldehyd oder Methacrolein. Im übrigen gelten diesbezüglich die in der DE-A 19740253 und DE-A 19740252 gemachten Angaben.

Wie beschrieben kann die Polymerisationsinhibierung beim erfindungsgemäßen Verfahren auch so vorgenommen werden, daß man am Kopf der Kolonne eine wäßrige MEHQ-Lösung aufgibt und in den Mittelteil der Kolonne eine Lösung von PTZ in Acrylsäure zusetzt. Der Sauerwasserquench wird dann ebenfalls mittels wäßriger MEHQ-Lösung stabilisiert.

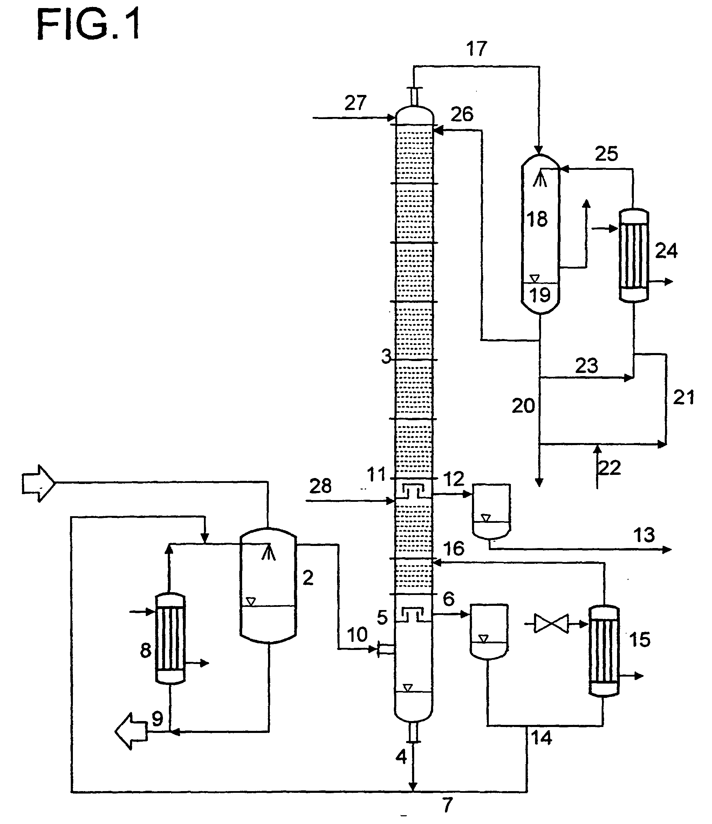

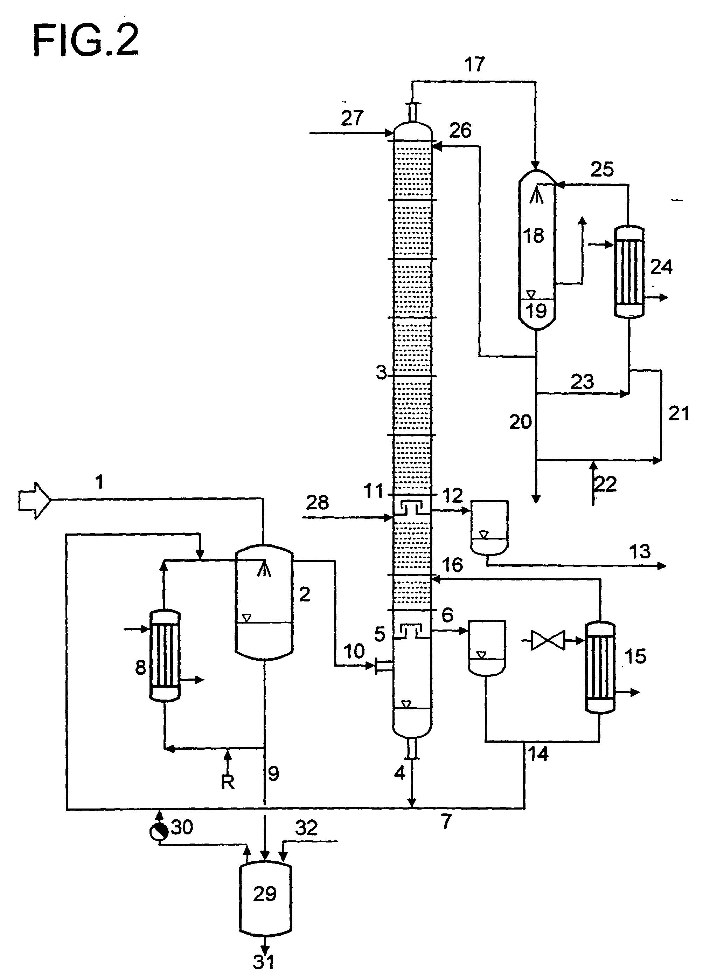

Es überrascht, daß die erfindungsgemäße Integration der Rückspaltung die Standzeit des Aufarbeitungsverfahrens so gut wie nicht beeinträchtigt Fig. 2 zeigt eine schematische Abbildung der erfindungsgemäßen Verfahrensweise. Die Bedeutung der Ziffern in Fig. 2 weisen das Vergleichsbeispiel und die Beispiele aus.

### Beispiele

Vergleichsbeispiel (die in diesem Beispiel verwendeten Bezugsziffern beziehen sich auf die dieser Schrift beiliegende Figur 1)

Aus einer heterogen katalysierten Gasphasenoxidation wurde ein eine Temperatur von 270°C aufweisendes Produktgasgemisch (1) der nachfolgenden Zusammensetzung erhalten:

| | |
|---|---|
| 11,5 Gew.-% | Acrylsäure, |
| 0,3 Gew. -% | Essigsäure, |
| 30 Gew.ppm | Propionsäure, |
| 0,09 Gew. -% | Maleinsäureanhydrid, |
| 0,01 Gew.-% | Acrolein, |
| 0,1 Gew.-% | Formaldehyd, |
| 30 Gew.ppm | Furfural, |
| 0,001 Gew.-% | Benzaldehyd, |
| 0,3 Gew.-% | Propen, |
| 3,4 Gew.-% | Sauerstoff, |
| 5,3 Gew.-% | Wasser, |
| 1,7 Gew.-% | Kohlenoxide, und als Restmenge N₂ |

Das Produktgasgemisch (3600 g/h) wurde in einem Sprühkühler (2) auf eine Temperatur von 136°C abgekühlt. Als Sprühflüssigkeit (Quenchflüssigkeit 1) wurden 750g/h (7) von insgesamt 7000g/h über den Fangboden (5) (mit einer Temperatur von 100°C) aus der Trennkolonne (3) entnommener Schwersiederfraktion (6) verwendet (Sumpfflüssigkeit 4 trat nicht auf). Über den mit Wärmeträgeröl betriebenen Rohrbündelwärmetauscher (8) wurde die Sprühflüssigkeit im Kreis geführt. 40 g/h der Quenchflüssigkeit 1 wurden dem Kreislauf als Auslaß kontinuierlich entnommen (9).

Das auf eine Temperatur von 136°C abgekühlte Produktgasgemisch wurde unterhalb des Fangbodens (5) der Trennkolonne zugeführt (10).

Die Kolonne war eine Bodenkolonne mit 25 Dual-flow- und 50 Glokkenböden (1 Glocke pro Boden). Der Bodendurchmesser betrug 49 mm. Die Dual-flow-Böden wiesen 6 Löcher pro Boden auf. Der Lochdurchmesser der ersten fünf Dual-flow-Böden betrug 9,5 mm. Die darauffolgenden 10 Böden hatten einen Lochdurchmesser von 9 mm und der Lochdurchmesser der letzten 5 Dual-flow-Böden betrug 8,7 mm. Der Boden obenhalb von Boden 15 war als weiterer Fangboden (11) gestaltet. Über ihm wurden 1800g/h einer eine Temperatur von 97°C aufweisenden Rohacrylsäure (12) enthaltend

| | |
|---|---|
| Acrylsäure | 97,3 Gew.-% |
| Essigsäure | 0,8 Gew.-% |
| Propionsäure | 600 Gew.ppm |
| Furfural | 700 Gew.ppm |
| Maleinsäureanhydrid | 40 Gew.ppm |
| Benzaldehyd | 200 Gew.ppm |
| Wasser | 1,3 Gew.-% |

abgezogen und einem Suspensionskristaller (13) zugeführt. Ein Teil (6250 g/h) der entnommenen Schwersiederfraktion (14) wurde in einem mit Wärmeträgeröl betriebenen Rohrbündelwärmetauscher auf 105°C erwärmt und auf den 5ten Boden in die Kolonne rückgeführt (16).

Der Kristaller war ein Rührbehälter (3 l Innenvolumen) mit Wendelrührer. Die Kristallisationswärme wurde über den Doppelmantel des Behälters abgeführt. Die-Gleichgewichtstemperatur der Lösung betrug 9,7°C. Die bei der Kristallisation erzeugte Suspension (Feststoffgehalt ca. 25 Gew.-%) wurde auf einer Zentrifuge bei 2000 U/min (Zentrifugendurchmesser 300 mm) und einer Schleuderzeit von 3 min diskontinuierlich in Kristalle und Mutterlauge getrennt. Die Kristalle wurden anschließend mit aufgeschmolzenem (zuvor gewaschenem) Kristallisat (80 g) 20 sec lang bei 2000 U/min gewaschen. Die Mutterlauge wurde zusammen mit der Waschflüssigkeit auf den 15ten Boden in die Trennkolonne rückgeführt (28).

Die Analyse der aufgeschmolzenen Reinkristalle (370 g/h) ergab folgende Gehalte:

| | |
|---|---|
| Acrylsäure | 99,5 Gew.-% |
| Essigsäure | 0,3 Gew.-% |
| Propionsäure | 200 Gew.ppm |
| Maleinsäureanhydrid | 20 Gew.ppm |
| Furfural | 20 Gew.ppm |
| Benzaldehyd | 5 Gew.ppm |
| Wasser | 0,06 Gew. -% |

Am Kopf der Kolonne wurde ein gasförmiges Gemisch (17) entnommen und im Sprühkühler (18) einer Partialkondensation unterworfen. 480 g/h des dabei anfallenden Sauerwassers wurden am Kopf der Kolonne mit einer Temperatur von 30°C in selbige zurückgeführt (26). 220 g/h des Sauerwassers wurden kontinuierlich entnommen. 90 g/h des entnommenen Sauerwassers wurden mit MEHQ (22) versetzt und als 0,5 gew. -%ige wässrige Stabilisatorlösung (21) gemeinsam mit der Restmenge des Sauerwassers (23) über den wassergekühlten Rohrbündelwärmetauscher (24) auf 18°C abgekühlt als Sprühflüssigkeit (25) verwendet. Mit einem anderen Teil des entnommenen Sauerwassers (ersatzweise kann hier auch eine entsprechende Menge Frischwasser verwendet werden) wurde eine 0,5 gew.-%ige wäßrige Lösung von 4-Hydroxy-TEMPO hergestellt, die in einer Menge von 18 g/h mit einer Temperatur von 20 bis 30°C auf dem 75ten Boden der Trennkolonne zugeführt wurde (27).

Die beschriebene Trennvorrichtung konnte 40 Tage ohne nennenswerte Polymerisatbildung betrieben werden.

### Beispiel 1

In einem beheizbaren Rührreaktor (Doppelmantel) mit 1 l Innenvolumen wurden 800 g des Auslaß der Quenchflüssigkeit 1 aus dem Verlgeichsbeispiel mit 1 Gew.-% Na₂CO₃ (bezogen auf das Gewicht der Auslaßmenge versetzt und bei einem Druck von 300 mbar auf 180°C erhitzt. Die Spaltprodukte wurden gasförmig über eine mit Raschigringen (5 mm) gefüllte Kolonne (Füllhöhe: 30 cm) kontinuierlich abgetrennt und ohne Zusatz von Polymerisationsinhibitor kondensiert. Innerhalb von 3,5 h Stunden wurden 730 g Spaltprodukt kondensiert. Dies entspricht einer Rückgewinnungsrate von ca. 90 Gew.-%. Entsprechend der gaschromatographischen Analyse bestand das Kondensat außer aus Acrylsäure und Inhibitor noch aus Diacrylsäure (ca. 1 Gew.-%), 0,15 Gew.-% Benzaldehyd und ca. 0,08 Gew.-% Furfurol.

### Beispiel 2

Es wurde wie in Beispiel 1 verfahren, doch an Stelle von 1 Gew. -% Na₂CO₃ wurde 1 Gew.-% Dodecylbenzolsulfonsäure als Spaltkatalysator eingesetzt. Innerhalb von 4,75 h wurden 495 g Spaltprodukt einer ähnlichen Zusammensetzung wie in Beispiel 1 kondensiert. Dies entspricht einer Rückgewinnungsrate von ca. 61 Gew.-%.

### Beispiel 3

Ein aus Glas bestehender Umlaufreaktor (29), der mit einer Heizkerze beheizt wurde, wurde mit 250 g des Auslaß der Quenchflüssigkeit 1 aus dem Vergleichsbeispiel gefüllt und erhitzt. Zusätzlich zu dem in den 250 g Auslaß der Quenchflüssigkeit 1 enthaltenen 4-OH-TEMPO wurden zum Zweck der co-Stabilisierung 2000 gew.ppm Phenothiazin zugesetzt. Die Spalttemperatur betrug 182°C und der Spaltdruck lag bei 500 mbar. Eine Spaltkatalysatorzugabe (32) erfolgte nicht. Standgeregelt wurden dem wie beschrieben gefüllten Umlaufreaktor 155 g/h Auslaß der Quenchflüssigkeit 1 (9) zugeführt. Die Spaltprodukte wurden dampfförmig über einen dem Reaktor aufgesetzten Spritzschutz abgetrennt und in einer Menge von 113 g/h (dies entspricht einer Rückgewinnungsrate von 73 Gew.-%) ohne Zugabe von Polymerisationsinhibitor kondensiert (30). Das Kondensat bestand neben Acrylsäure und Inhibitor noch aus ca. 3,2 Gew.-% Diacrylsäure, 0,06 Gew.-% Benzaldehyd, 0,3 Gew.-% Essigsäure und 0,03 Gew.-% Propionsäure. 40 g/h Spaltrückstand wurden dem Spaltreaktor kontinuierlich entnommen (31) und entsorgt.

### Beispiel 4

Wie das Vergleichsbeispiel, der Sprühflüssigkeit wurden jedoch zusätzlich 30 g/h des auf 100°C abgekühltem Rückspaltkondensats aus Beispiel 3 zugeführt (die Zugabestelle lag kurz vor der Sprühdüse). In entsprechender Weise betrug die Menge der gewonnenen Reinkristalle ca. 400 g/h.

Die Rückführung des Spaltkondensats hatte innerhalb von 40 Betriebstagen der beschriebenen Trennvorrichtung keinen sichtbaren Einfluß auf die Polymerisatbildung. Als alternative Zugabestelle für das Rückspaltkondensat kommt auch die mit R gekennzeichnete Stelle in Fig. 2 in Betracht.

## Patentansprüche

1. Verfahren zur Herstellung von Acrylsäure, bei dem man durch heterogen katalysierte Gasphasen-Partialoxidation von Propan oder Propen und/oder Acrolein mit molekularem Sauerstoff an im festen Aggregatzustand befindlichen Katalysatoren bei erhöhter Temperatur ein Acrylsäure enthaltendes heißes Produktgasgemisch erzeugt, die Temperatur des heißen, Acrylsäure enthaltenden Produktgasgemisches durch direkte Kühlung mit einer Quenchflüssigkeit 1 verringert, den dabei nicht verdampfenden Teil der Quenchflüssigkeit 1, gegebenenfalls über einen indirekt kühlenden Wärmetauscher, im Kreis führt und als Auslaß einen Teil der nicht verdampften Quenchflüssigkeit 1 aus diesem Kreislauf ausschleust, anschließend das abgekühlte Produktgasgemisch in eine mit trennwirksamen Einbauten ausgerüstete Kolonne leitet, innerhalb der Kolonne in sich selbst aufsteigen läßt und dabei fraktioniert kondensiert, wobei im Seitenabzug eine rohe Acrylsäure und über den Kolonnensumpf und/oder über einen in Sumpfnähe gelegenen Seitenabzug Acrylsäure-Oligomere enthaltende Sumpfflüssigkeit und/oder Schwersiederfraktion entnommen und als Quenchflüssigkeit 1 verwendet wird, **dadurch gekennzeichnet, daß** man den Auslaß der Quenchflüssigkeit 1 einem Spaltgefäß zuführt und in selbigem die im Auslaß der Quenchflüssigkeit 1 enthaltenen Acrylsäure-Oligomere bei erhöhter Temperatur in Acrylsäure rückspaltet, dabei gasförmig aus der Flüssigphase entweichende Acrylsäure kondensiert und das resultierende Kondensat in den Kreislauf der Quenchflüssigkeit 1 einspeist.

2. Verfahren nach Anspruch 1, **dadurch gekennzeichnet, daß** die Rückspaltung im Beisein eines anorganischen Salzes, dessen Zusatz zu einer wäßrigen Lösung einer starken Brönsted-Säure den pH-Wert der wäßrigen Lösung ins alkalische verschiebt, durchgeführt wird.

3. Verfahren nach Anspruch 2, **dadurch gekennzeichnet, daß** als anorganisches Salz wenigstens eines aus der Gruppe umfassend NaOH, Na₂CO₃, NaHCO₃, KOH, K₂CO₃, KHCO₃, LiOH, Li₂CO₃ und CaCO₃ verwendet wird.

4. Verfahren nach einem der Ansprüche 1 bis 3, **dadurch gekennzeichnet, daß** die Rückspaltung bei einer Temperatur von 130 bis 250°C durchgeführt wird.

5. Verfahren nach einem der Ansprüche 1 bis 4, **dadurch gekennzeichnet, daß** die Rückspaltung bei einem Druck von 25 bis 600 mbar durchgeführt wird.

6. Verfahren nach einem der Ansprüche 1 bis 5, **dadurch gekennzeichnet, daß** innerhalb der zur fraktionierten Kondensation verwendeten Kolonne 4-OH-TEMPO als Polymerisationsinhibitor mitverwendet wird.

## Claims

1. A process for the preparation of acrylic acid, in which an acrylic acid-containing hot product gas mixture is produced by gas-phase partial oxidation of propane or propene and/or acrolein with molecular oxygen under heterogeneous catalysis over catalysts present in the solid state at elevated temperature, the temperature of the hot acrylic acid-containing product gas mixture is reduced by direct cooling by means of a quench liquid 1, the unvaporized part of the quench liquid 1 is circulated, if required via a heat exchanger providing indirect cooling, and a part of the unvaporized quench liquid 1 is removed as a purge from the circulation, and the cooled product gas mixture is then passed into a column equipped with baffles having separation activity, allowed to ascend into itself inside the column and subjected to fractional condensation, a crude acrylic acid being removed at the side take-off and bottom liquid containing acrylic acid oligomers and/or a high boiler fraction being removed via the bottom of the column and/or via a side take-off in the vicinity of the bottom and being used as quench liquid 1, wherein the purged quench liquid 1 is fed to a cleavage vessel, the acrylic acid oligomers contained in the purged quenched liquid 1 are cleaved in said vessel at elevated temperatures to give acrylic acid, acrylic acid escaping in gaseous form from the liquid phase is condensed and the resulting condensate if fed into the circulation of the quench liquid 1.

2. A process as claimed in claim 1, wherein the cleavage is carried out in the presence of an inorganic salt, the addition of which to an aqueous solution of a strong Brönsted acid shifts the pH of the aqueous solution toward the alkaline.

3. A process as claimed in claim 2, wherein the inorganic salt used is at least one from the group consisting of NaOH, Na₂CO₃, NaHCO₃, KOH, K₂CO₃, KHCO₃, LiOH, Li₂CO₃ and CaCO₃.

4. A process as claimed in any of claims 1 to 3, wherein the cleavage is carried out at from 130 to 250°C.

5. A process as claimed in any of claims 1 to 4, wherein the cleavage is carried out at from 25 to 600 mbar.

6. A process as claimed in any of claims 1 to 5, wherein 4-OH-TEMPO is present as a polymerisation inhibitor inside the column used for the fractional condensation.

## Revendications

1. Procédé de préparation d'acide acrylique, dans lequel on produit, par oxydation partielle en phase gazeuse à catalyse hétérogène de propane ou de propène et / ou d'acroléine par de l'oxygène moléculaire sur des catalyseurs se trouvant à l'état d'agrégats solides, à température élevée, un mélange de produits gazeux chaud contenant de l'acide acrylique, on abaisse la température du mélange de produits gazeux chaud contenant de l'acide acrylique par refroidissement direct au moyen d'un liquide de refroidissement 1, on guide en circuit la partie non évaporée du liquide de refroidissement 1, éventuellement via un échangeur de chaleur à refroidissement indirect et on soutire comme décharge une partie du liquide de refroidissement 1 non évaporé hors de ce circuit, en envoie ensuite le mélange de produits gazeux refroidi dans une colonne équipée d'éléments encastrés à action de séparation, on le laisse monter de lui-même dans la colonne et on opère ainsi une condensation fractionnée, en prélevant d'un soutirage latéral un acide acrylique brut et par le fond de colonne et / ou un soutirage latéral placé à proximité du fond de colonne, un liquide de queue contenant des oligomères d'acide acrylique et / ou une fraction à haut point d'ébullition que l'on utilise comme liquide de refroidissement 1, **caractérisé en ce que** la décharge de liquide de refroidissement 1 est envoyée à un réservoir de décomposition, dans lequel les oligomères d'acide acrylique contenus dans la sortie de liquide de refroidissement se décomposent à nouveau en acide acrylique à température élevée, on condense l'acide acrylique se dégageant alors sous forme gazeuse de la phase liquide, et on alimente le condensat résultant dans le circuit du liquide de refroidissement 1.

2. Procédé selon la revendication 1, **caractérisé en ce que** la redécomposition est entreprise en présence d'un sel inorganique, dont l'addition à une solution aqueuse d'un acide fort de Brönsted déplace le pH de la solution aqueuse dans la plage alcaline.

3. Procédé selon la revendication 2, **caractérisé en ce que** l'on utilise comme sel inorganique au moins un sel du groupe comprenant NaOH, Na₂CO₃, NaHCO₃, KOH, K₂CO₃, KHCO₃, LiOH, Li₂CO₃ et CaCO₃.

4. Procédé selon l'une des revendications 1 à 3, **caractérisé en ce que** la redécomposition est entreprise à une température de 130 à 250°C.

5. Procédé selon l'une des revendications 1 à 4, **caractérisé en ce que** la redécomposition est entreprise à une pression de 25 à 600 mbar.

6. Procédé selon l'une des revendications 1 à 5, **caractérisé en ce que** l'on utilise conjointement du 4-OH-TEMPO comme inhibiteur de polymérisation dans la colonne utilisée pour la condensation fractionnée.
